# EUROPEAN PATENT APPLICATION

(11) **EP 4 512 442 A2**
(43) Date of publication of application: **26.02.2025**
(21) Application number: 24217771.5
(22) Date of filing: 25.06.2015
(51) Int. Cl.: A61M 5/142

(54) **FLUID CONDUIT ASSEMBLY WITH GAS TRAPPING FILTER IN THE FLUID FLOW PATH**

(30) Priority: 07.10.2014 US 201414508934
(62) Divisional of application: 21198501.5
(71) Applicant: MEDTRONIC MINIMED, INC., Northridge, CA 91325-1219 (US)
(72) Inventor: CHATTARAJ, Sarnath, Simi Valley, 93063 (US); DANG, Kiem H., Thousand Oaks, 91360 (US); GULATI, Poonam S., La Canada, 91011 (US); ZHANG, Guangping, Calabasas, 91302 (US)
(74) Representative: Zimmermann & Partner Patentanwälte mbB

(57) **Abstract**

A fluid delivery system and a fluid conduit assembly suitable for use with the system are disclosed herein. The system includes a fluid infusion pump and a fluid conduit assembly coupled to the pump to deliver medication fluid to a user. The fluid conduit assembly includes a structure defining a flow path for the medication fluid, and a gas trapping filter coupled to the structure and positioned in the flow path. The gas trapping filter functions to filter particulates from the medication fluid and retain gas bubbles from the medication fluid.

## Description

### CROSS-REFERENCE TO RELATED APPLICATIONS

This PCT application claims the benefit of and claims priority to: United States Patent Application Serial Number 14/508,934, filed October 7, 2014.

### TECHNICAL FIELD

Embodiments of the subject matter described herein relate generally to fluid infusion devices for delivering a medication fluid to the body of a user. More particularly, embodiments of the subject matter relate to the use of a gas trapping filter in the medication fluid flow path.

### BACKGROUND

Certain diseases or conditions may be treated, according to modern medical techniques, by delivering a medication fluid or other substance to the body of a patient, either in a continuous manner or at particular times or time intervals within an overall time period. For example, diabetes is commonly treated by delivering defined amounts of insulin to the patient at appropriate times. Some common modes of providing insulin therapy to a patient include delivery of insulin through manually operated syringes and insulin pens. Other modern systems employ programmable fluid infusion devices (e.g., continuous insulin infusion devices such as insulin pumps) to deliver controlled amounts of insulin or other drugs to a patient.

A fluid infusion device suitable for use as an insulin pump may be realized as an external device or an implantable device, which is surgically implanted into the body of the patient. External fluid infusion devices include devices designed for use in a generally stationary location (for example, in a hospital or clinic), and devices configured for ambulatory or portable use (to be carried by a patient). External fluid infusion devices may establish a fluid flow path from a fluid reservoir to the patient via, for example, a suitable hollow tubing. The hollow tubing may be connected to a hollow fluid delivery needle that is designed to pierce the patient's skin to deliver an infusion fluid to the body. Alternatively, the hollow tubing may be connected directly to the patient's body through a cannula or set of micro-needles.

It is desirable to reduce the amount of air bubbles in a medication fluid before delivering the fluid to the patient. Small bubbles may be introduced into the medication fluid during a reservoir filling operation, for example, when the fluid reservoir is filled from a vial using a syringe. Although patients are instructed to eliminate air from a filled reservoir, some micro bubbles may remain.

Accordingly, it is desirable to have an assembly, system, or component that is designed to mitigate the effects of air bubbles within a medication fluid flow path. In addition, it is desirable to have an assembly, system, or component that reduces the presence of air bubbles in a fluid flow path while also filtering particulates and/or unwanted substances from the medication fluid. Furthermore, other desirable features and characteristics will become apparent from the subsequent detailed description and the appended claims, taken in conjunction with the accompanying drawings and the foregoing technical field and background.

### BRIEF SUMMARY

Disclosed herein is a fluid conduit assembly for delivery of a medication fluid. An exemplary embodiment of the fluid conduit assembly includes a structure defining a flow path for the medication fluid and a gas trapping filter coupled to the structure. The gas trapping filter is positioned in the flow path to filter particulates from the medication fluid and retain gas bubbles from the medication fluid.

A fluid delivery system is also disclosed herein. An exemplary embodiment of the system includes: a fluid infusion pump to provide a medication fluid; a fluid conduit assembly coupled to the fluid infusion pump; and a gas trapping filter. The fluid conduit delivers the medication fluid to a user, and the fluid conduit assembly defines a flow path for the medication fluid. The gas trapping filter is positioned in the flow path to filter particulates from the medication fluid and retain gas bubbles from the medication fluid.

Also disclosed herein is a fluid conduit assembly for delivery of a medication fluid. An exemplary embodiment of the fluid conduit assembly includes a body section to receive a fluid reservoir, and a flow path defined in the body section. The flow path carries fluid from the fluid reservoir when the body section is coupled to the fluid reservoir. The fluid conduit assembly also has a length of tubing extending from the body section and in fluid communication with the flow path. The length of tubing carries fluid from the body section during a fluid delivery operation. The fluid conduit assembly also has a partially or predominantly hydrophilic gas trapping filter positioned in the flow path to filter particulates from the medication fluid and retain gas bubbles from the medication fluid.

One embodiment of the present invention comprises a cap for a reservoir of a medicinal fluid which provides a fluid flow path for the medicinal fluid to tubing used to deliver said fluid to a user; said cap comprising: a lower body section configured to be able to engage with the reservoir and having an axial bore to conduct the medicinal fluid from the reservoir; an upper body section having an axial bore for connection at one end to the tubing, the axial bore of the upper body section being in fluid communication with the axial bore of the lower body section to receive the medication fluid via an internal chamber; wherein a gas trapping filter is housed within the internal chamber and configured to retain gas bubbles thereby preventing said gas from leaving the internal chamber.

The gas trapping filter may be adapted to have one or more of the following additional functions: a. filter out particulates; b. adsorb or absorb silicone oil; c. form a depot of a drug such that the drug is released into the medicinal fluid as it passes through. Particularly where the gas trapping filter is adapted to filter out particulates, it can have a pore size within the range of 0.45 to 5.00 microns. The gas trapping filter may comprise a hydrophilic sponge, felt or fiber composite material. If the gas trapping filter is of felt it preferably has a pore size in the range 1-100 micrometres, more preferably 20 to 40 micrometres. If the gas trapping filter is of sponge it preferably has a pore size in the range 20 micrometres to 1 millimetre.

The lower body section and the upper body section may be integral with one another. Whether or not the body sections are integral the lower body section may include an upstream hollow needle disposed axially within the axial bore and in fluid communication with the internal chamber upstream of the gas trapping filter for piecing a septum in the reservoir and conducting the medicinal fluid therefrom to the internal chamber. The gas trapping filter may comprise a discrete or an integrally formed cylindrical component axially disposed in the internal chamber and having a ratio of diameter to height of greater than unity, preferably 5:1 or greater. If discrete the gas trapping filter may be is held in the internal chamber by a first annular flange forming part of the lower body section, and second annular flange forming part of the upper body section, at least one of said annular flanges also sealing against margins of respective major surfaces of the gas trapping filter ensuring that the flow of medication fluid is through a central region of the filter.

On the other hand, the gas trapping filter may comprise a discrete cylindrical component axially disposed in the internal chamber and having a ratio of diameter to height of less than or equal to unity. In this case there may be provided a hydrophilic membrane separating the internal chamber from the bore of the upper body section. In all cases the upper body section may be a manual grip radially outwardly from the gas trapping filter enabling the cap to readily be turned by hand about its longitudinal axis. Such a cap can form part of a disposible fluid delivery assembly comprising a reservoir of the medicinal fluid, wherein the cap is further being provided with thread or bayonet fitting for detachably mounting the reservoir in an infusion pump, whereby the assembly can be detached for disposal by turning the cap via said manual grip.

In a further embodiment, the invention provides a fluid delivery system for delivering a medicinal fluid to a user comprising: an infusion pump including a reservoir of the medicinal fluid, said reservoir having a cap as described above; a length of tubing connected at a proximal end to the axial bore of the upper body section of the cap, and at a distal end to an infusion unit having a canula to enable fluid from the reservoir to be infused into the body of the user during operation of the pump.

The present invention according to a further embodiment provides a fluid connector assembly for a medication fluid comprising: a first connector having a bore for connection at one end to an upstream tubing; a second connector having a bore for connection at one end to a downstream tubing; the first and second connectors being detachably couplable, such that when coupled the respective bores are in fluid communication allowing flow of the medication fluid from the upstream tubing to the downstream tubing via the said coupled bores; wherein one of the first and second connectors contains a hollow needle and the other contains a septum, and when the first and second connectors are coupled the needle pierces the septum thereby providing the said fluid communication between the bores of the said respective connectors; a gas trapping filter disposed in the bore of the second connector to retain gas bubbles and hinder such bubbles from traveling with the medication fluid into the downstream tubing. The gas trapping filter is adapted to have one or more of the following additional functions: a. filter out particulates; b. adsorb or absorb silicone oil; c. form a depot of a drug such that the drug is released into the medicinal fluid as it passes through. The gas trapping filter may is mold inside the bore of the second connector forming an integral component therewith. The fluid connector assembly may further include a second gas trapping filter disposed in the bore of the first connector to detain gas bubbles in the medication fluid entering the fluid connector assembly from the upstream tubing. In this case the second gas trapping filter may be adapted to have one or more of the following additional functions: a. filter out particulates; b. adsorb or absorb silicone oil; c. form a depot of a drug such that the drug is released into the medicinal fluid as it passes through. In all of embodiments the gas trapping filter may be of a predominantly hydrophilic material optionally exhibiting up to 50% hydrophobicity.

This summary is provided to introduce a selection of concepts in a simplified form that are further described below in the detailed description. This summary is not intended to identify key features or essential features of the claimed subject matter, nor is it intended to be used as an aid in determining the scope of the claimed subject matter.

### BRIEF DESCRIPTION OF THE DRAWINGS

A more complete understanding of the subject matter may be derived by referring to the detailed description and claims when considered in conjunction with the following figures, wherein like reference numbers refer to similar elements throughout the figures.
FIG. 1 is a simplified block diagram representation of an embodiment of a fluid delivery system;
FIG. 2 is a plan view of an exemplary embodiment of a fluid delivery system that includes a fluid infusion device and an infusion set;
FIG. 3 is a perspective view of an exemplary embodiment of a fluid delivery system that includes a fluid infusion device designed to be affixed to the skin of the user;
FIG. 4 is a schematic representation of a portion of a fluid conduit assembly;
FIG. 5 is an exploded and partially phantom view of a connector assembly suitable for use with a fluid conduit;
FIG. 6 is an exploded perspective view of an embodiment of a fluid conduit assembly that is realized as a cap for a fluid reservoir; and
FIG. 7 is an exploded perspective view of another embodiment of a fluid conduit assembly that is realized as a cap for a fluid reservoir.

### DETAILED DESCRIPTION

The following detailed description is merely illustrative in nature and is not intended to limit the embodiments of the subject matter or the application and uses of such embodiments. As used herein, the word "exemplary" means "serving as an example, instance, or illustration." Any implementation described herein as exemplary is not necessarily to be construed as preferred or advantageous over other implementations. Furthermore, there is no intention to be bound by any expressed or implied theory presented in the preceding technical field, background, brief summary or the following detailed description.

The subject matter described here relates to certain assemblies, components, and features of a fluid infusion system of the type used to treat a medical condition of a patient. The fluid infusion system is used for infusing a medication fluid into the body of a user. The non-limiting examples described below relate to a medical device used to treat diabetes (more specifically, an insulin pump), although embodiments of the disclosed subject matter are not so limited. Accordingly, the medication fluid is insulin in certain embodiments. In alternative embodiments, however, many other fluids may be administered through infusion such as, but not limited to, disease treatments, drugs to treat pulmonary hypertension, iron chelation drugs, pain medications, anti-cancer treatments, medications, vitamins, hormones, or the like. Moreover, the gas trapping filter described below could be utilized in the context of other fluid delivery systems if so desired.

For the sake of brevity, conventional features and technologies related to infusion system operation, insulin pump and/or infusion set operation, and other functional aspects of the fluid infusion system (and the individual operating components of the system) may not be described in detail here. Examples of infusion pumps and/or related pump drive systems used to administer insulin and other medications may be of the type described in, but not limited to, United States patent numbers: 4,562,751; 4,678,408; 4,685,903; 5,080,653; 5,505,709; 5,097,122; 6,485,465; 6,554,798; 6,558,351; 6,659,980; 6,752,787; 6,817,990; 6,932,584; and 7,621,893; which are herein incorporated by reference.

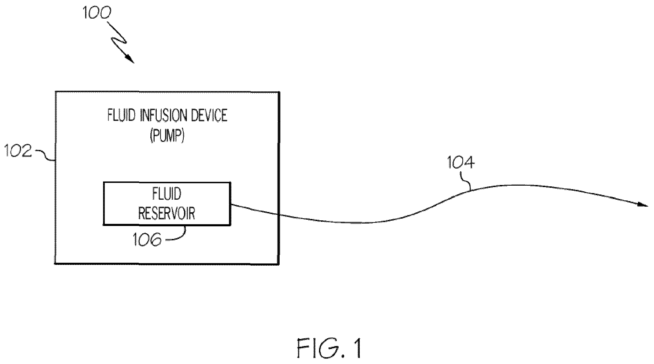

FIG. 1 is a simplified block diagram representation of an embodiment of a fluid delivery system 100, which can be utilized to administer a medication fluid such as insulin to a patient. The fluid delivery system 100 includes a fluid infusion device 102 (e.g., an infusion pump) and a fluid conduit assembly 104 that is coupled to, integrated with, or otherwise associated with the fluid infusion device 102. The fluid infusion device 102 includes a fluid reservoir 106 or an equivalent supply of the medication fluid to be administered. The fluid infusion device 102 is operated in a controlled manner to deliver the medication fluid to the user via the fluid conduit assembly 104. Although not depicted in FIG. 1, the fluid delivery system 100 also includes a gas trapping filter that is positioned in the fluid flow path. In certain embodiments, the gas trapping filter is located within the fluid flow path defined by the fluid conduit assembly 104.

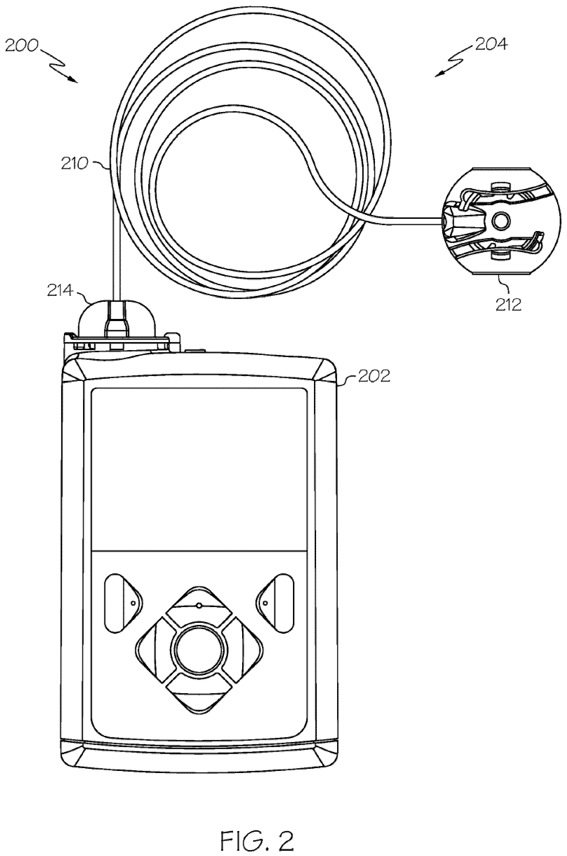

The fluid infusion device 102 may be provided in any desired configuration or platform. In accordance with one non-limiting embodiment, the fluid infusion device is realized as a portable unit that can be carried or worn by the patient. In this regard, FIG. 2 is a plan view of an exemplary embodiment of a fluid delivery system 200 that includes a portable fluid infusion device 202 and a fluid conduit assembly that takes the form of an infusion set component 204. For this particular embodiment, the infusion set component 204 can be coupled to the fluid infusion device 202 as depicted in FIG. 2. The fluid infusion device 202 accommodates a fluid reservoir (hidden from view in FIG. 2) for the medication fluid to be delivered to the user.

The illustrated embodiment of the infusion set component 204 includes, without limitation: a tube 210; an infusion unit 212 coupled to the distal end of the tube 210; and a connector assembly 214 coupled to the proximal end of the tube 210. The fluid infusion device 202 is designed to be carried or worn by the patient, and the infusion set component 204 terminates at the infusion unit 212 such that the fluid infusion device 202 can deliver fluid to the body of the patient via the tube 210. The fluid infusion device 202 may leverage a number of conventional features, components, elements, and characteristics of existing fluid infusion devices. For example, the fluid infusion device 202 may incorporate some of the features, components, elements, and/or characteristics described in United States Patent numbers 6,485,465 and 7,621,893, the relevant content of which is incorporated by reference herein.

The infusion set component 204 defines a fluid flow path that fluidly couples the fluid reservoir to the infusion unit 212. The connector assembly 214 mates with and couples to the neck region of the fluid reservoir, establishing the fluid path from the fluid reservoir to the tube 210. The connector assembly 214 (with the fluid reservoir coupled thereto) is coupled to the housing of the fluid infusion device 202 to seal and secure the fluid reservoir inside the housing. Thereafter, actuation of the fluid infusion device 202 causes the medication fluid to be expelled from the fluid reservoir, through the infusion set component 204, and into the body of the patient via the infusion unit 212 at the distal end of the tube 210. Accordingly, when the connector assembly 214 is installed as depicted in FIG. 2, the tube 210 extends from the fluid infusion device 202 to the infusion unit 212, which in turn provides a fluid pathway to the body of the patient. For the illustrated embodiment, the connector assembly 214 is realized as a removable reservoir cap (or fitting) that is suitably sized and configured to accommodate replacement of fluid reservoirs (which are typically disposable) as needed.

Although not visible in FIG. 2 the fluid delivery system 200 includes a gas trapping filter in the connector assembly 214 preferably immediately downstream of the reservoir. This traps any bubbles which may inadvertently enter the fluid pathway when the fluid reservoir is replaced. The filter itself can, but need not be, itself independently replaceable such that when the reservoir is replaced the filter can be removed from the cap / connector assembly 214 and replaced separately.

Another gas trapping filter may be included in the infusion unit 212 just downstream of the point when the tubing 210 enters the infusion unit, i.e. beyond the distal end of the tubing. With this second filter any gas that manages to pass the first filter is prevented from entering the patient. It is also envisaged that one of the filters be omitted. Either or both filters may be constructed to act as a particulate filter. Accumulated particles in the filter are disposed of by filter replacement.

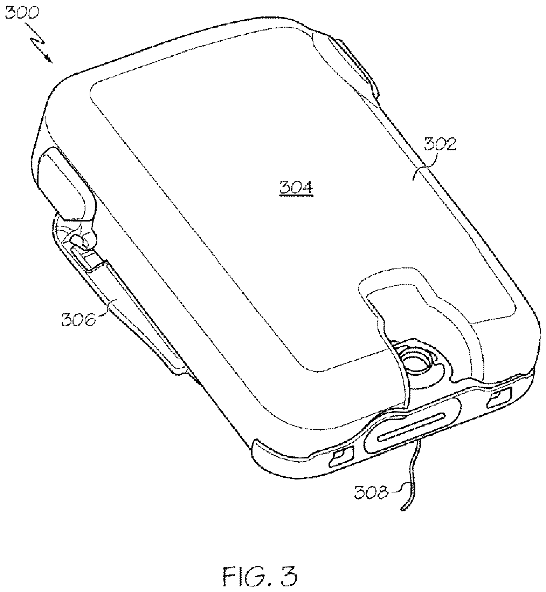

FIG. 3 is a perspective view of another exemplary embodiment of a fluid delivery system 300 that includes a fluid infusion device 302 designed to be affixed to the skin of the user. The fluid infusion device 302 includes two primary components that are removably coupled to each other: a durable housing 304; and a base plate 306. The fluid infusion device 302 also includes or cooperates with a removable/replaceable fluid reservoir (which is hidden from view in FIG. 3). For this particular embodiment, the fluid reservoir mates with, and is received by, the durable housing 304. In alternate embodiments, the fluid reservoir mates with, and is received by, the base plate 306.

The base plate 306 is designed to be temporarily adhered to the skin of the patient using, for example, an adhesive layer of material. After the base plate is affixed to the skin of the patient, a suitably configured insertion device or apparatus may be used to insert a fluid delivery needle or cannula 308 into the body of the patient. The cannula 308 functions as one part of the fluid delivery flow path associated with the fluid infusion device 302. In this regard, the cannula 308 may be considered to be one implementation of the fluid conduit assembly 104 shown in FIG. 1 (or a portion thereof).

FIG. 3 depicts the durable housing 304 and the base plate 306 coupled together. For this particular embodiment, the durable housing 304 contains, among other components, a drive motor, a battery, a threaded drive shaft for the fluid reservoir, one or more integrated circuit chips and/or other electronic devices (not shown). The durable housing 304 and the base plate 306 are cooperatively configured to accommodate removable coupling of the durable housing 304 to the base plate 306. The removable nature of the durable housing 304 enables the patient to replace the fluid reservoir as needed.

The fluid delivery systems 300 shown in FIG. 3 has a gas trapping filter in the fluid path leading from its internal reservoir (not shown) to the cannula 308. Typically, the gas trapping filter will be immediately upstream of the point where the cannula 308 exits through the base plate 306. Preferably the filter is replaceable at least when the reservoir is replaced. The gas trapping filter in the FIG. 3 arrangement may also be configured to be able to detain particulates.

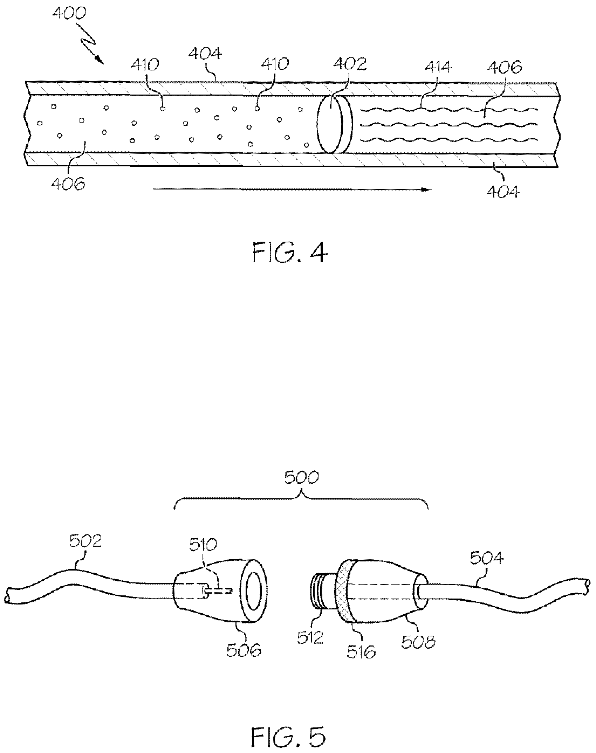

It is also envisaged that a gas trapping filter may be situated at an intermediate position in a length of fluid conduit. In this regard, FIG. 4 is a schematic representation of a portion of a fluid conduit assembly 400 having a gas trapping filter 402 positioned therein. It should be appreciated that the fluid conduit assembly 400 has been simplified for ease of illustration. In practice, the fluid conduit assembly 400 may be realized in any of the fluid delivery systems described here, and/or in other fluid delivery systems not specifically described in detail here. For example, the fluid conduit assembly 400 may be implemented as, or form a part of, a fluid infusion set, a connector assembly, a fluid reservoir, a fluid reservoir cap, a chamber or internal feature of an infusion pump, or the like.

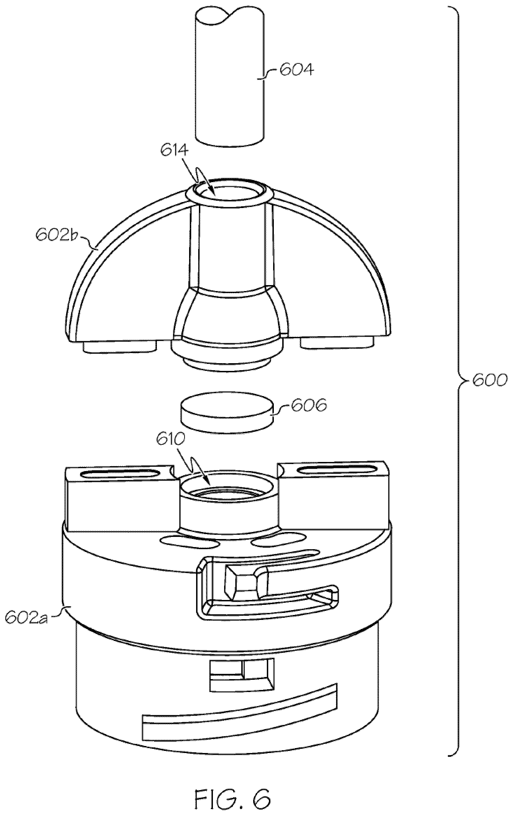

The fluid conduit assembly 400 is suitably configured to accommodate the delivery of a medication fluid such as insulin. The fluid conduit assembly 400 includes a structure 404 (or structures) defining a flow path 406 for the medication fluid. In FIG. 4, the structure 404 is depicted in cross section, and it resembles a tube. Alternatively, the structure 404 can be a section of a fluid connector (such as a two-part detachable connector), an internal feature of an infusion device, a portion of a fluid reservoir coupler, or the like. In certain embodiments, the structure 404 includes, forms a part of, or is realized as a reservoir cap for a fluid infusion device (see FIG. 6). In some embodiments, the structure 404 includes, forms a part of, or is integrated with an infusion set for a fluid infusion device. In this regard, the gas trapping filter 402 can be integrated with the delivery cannula hub or housing that is located at or near the downstream end of the infusion set. In yet other embodiments, the structure 404 includes, forms a part of, or is realized as a fluid connector, such as a LUER LOK fitting or connector. In certain embodiments, the structure 404 is implemented as a feature of the fluid infusion device. These and other deployments of the fluid conduit assembly 400 are contemplated by this disclosure, and the particular examples presented here are not intended to be limiting or exhaustive.

The flow path 406 is defined by the interior space of the structure 404. The gas trapping filter 402 may be coupled to the structure 404 and positioned in the flow path 406 such that the medication fluid passes through the gas trapping filter 402 during fluid delivery operations. FIG. 4 depicts a straightforward scenario where the gas trapping filter 402 physically obstructs the flow path 406, such that the medication fluid is not diverted around the gas trapping filter 402. In other embodiments, there can be additional fluid flow paths that allow some of the medication fluid to bypass the gas trapping filter 402.

The gas trapping filter 402 is formed from a suitable material, composition, or clement such that the medication fluid can easily pass through the gas trapping filter 402 during fluid delivery operations. The gas trapping filter 402 can be formed from a hydrophilic, semi-hydrophilic, partially hydrophilic, or predominantly hydrophilic material. Although a truly hydrophilic material may be ideal, the material used for the gas trapping filter 402 can be partially or predominantly hydrophilic while exhibiting some amount of hydrophobicity. In practice, the gas trapping filter 402 can exhibit up to fifty percent hydrophobicity without adversely impacting the desired performance. For example, the gas trapping filter 402 may include or be fabricated from a hydrophilic membrane, a hydrophilic sponge material, or a hydrophilic foam material. As explained below, the gas trapping filter 402 preferably also serves to filter particulates from the medication fluid during fluid delivery operations. Accordingly, the gas trapping filter 402 preferably has a pore size that is small enough to inhibit the flow of particulates. In certain embodiments, the pore size is within the range of about 0.45 to 5.00 microns, which is suitable for most medical applications. Non-limiting examples of suitable materials for the gas trapping filter 402 include: polyacrylate; polyurethane; nylon; cellulose acetate; polyvinyl alcohol; polyethelene foam; polyvinyl acetate; polyester fiber felt, polyester (PET); polysulfonc; polyethyl sulfone; collagen; polycaprolactone; or the like. It should be appreciated that the material or materials used to fabricate the gas trapping filter 402 can be treated to enhance the hydrophilic characteristics if so desired.

One function of the gas trapping filter 402 is to inhibit the downstream flow of air bubbles. Depending on the particular composition and configuration of the gas trapping filter 402, air bubbles 410 (depicted as small circles in the flow path 406 upstream of the gas trapping filter 402) can be blocked by the gas trapping filter 402 and/or retained within the gas trapping filter 402 as the liquid medication flows downstream. Thus, the gas trapping filter 402 may be realized as a gas impermeable membrane or material that also exhibits good hydrophilic properties. In some embodiments, the gas trapping filter 402 can be fabricated from material having micro-cavities formed therein for trapping and retaining gas bubbles from the medication fluid. FIG. 4 illustrates a scenario where the air bubbles 410 are removed from the medication fluid. Accordingly, no air bubbles 410 are present in the medication fluid that resides downstream from the gas trapping filter 402.

Another benefit of the gas trapping filter 402 relates to the volume accuracy of the fluid delivery system. In certain implementations, syringe pumps are calibrated to deliver a specified volume in response to a controlled mechanical actuation (e.g., movement of the syringe plunger in response to controlled rotation of an electric motor). Reducing or eliminating air from the fluid delivery path increases the accuracy of the volume calibrations.

In certain embodiments, the gas trapping filter 402 also serves to filter particulates from the medication fluid such that the particulate count of the downstream medication fluid is reduced. As mentioned above, the material used to fabricate the gas trapping filter 402 can be selected with a desired pore size to accommodate filtering of particulates having an expected size.

In some embodiments, the gas trapping filter 402 also serves to absorb and/or adsorb certain substances, chemicals, or suspended elements from the medication fluid. For example, the gas trapping filter 402 may include material that is configured or treated to absorb/adsorb lubricating or manufacturing oil that is associated with the manufacturing, assembly, or maintenance of one or more components of the fluid delivery system. In this regard, a fluid reservoir for insulin can be fabricated with a trace amount of silicone oil that serves as a lubricant for the plunger of the reservoir. Accordingly, the gas trapping filter 402 can include a material, layer, or treatment that reduces, traps, or otherwise removes some or all of the silicone oil from the medication fluid as it passes through the gas trapping filter 402.

In particular embodiments, the gas trapping filter 402 also serves as a drug depot during operation of the fluid delivery system. To this end, the gas trapping filter 402 can include a drug, medicine, chemical, or composition impregnated therein (or coated thereon, or otherwise carried by the gas trapping filter 402). A quantity of the drug is released into the medication fluid as the fluid flows through the gas trapping filter 402 during a fluid delivery operation. The wavy lines 414 in FIG. 4 schematically depict the drug after it has been released into the downstream medication fluid. In practice, the drug carried by the gas trapping filter 402 will eventually be depleted unless the gas trapping filter 402 or the fluid conduit assembly 400 is replaced before depletion. The drug carried by the gas trapping filter 402 can be selected to address the needs of the particular patient, fluid delivery system, medication fluid, etc. In accordance with the exemplary insulin infusion system described here, the gas trapping filter 402 is impregnated with a drug that treats the patient site to extend the useful life of the fluid infusion set. For example, the gas trapping filter 402 can be treated with an anticoagulant such as Heparin or Dextran. As another example, the gas trapping filter 402 can be impregnated or infused with an anti-proliferative drug such as Rapamycin. It should be appreciated that these examples are neither exhaustive nor restrictive, and that the gas trapping filter 402 can be impregnated, treated, or infused with any drug that may be appropriate and suitable for the particular medical condition, fluid delivery system, or application.

Similarly any of the gas trapping filters to be described in detail below with reference to figures 5-7 can also include a drug as described above.

Although FIG. 4 shows a single component that serves as the gas trapping filter 402, an embodiment of the fluid conduit assembly 400 can utilize a plurality of physically distinct elements that collectively function as the gas trapping filter 402. For example, the gas trapping filter 402 can be fabricated from different materials that are selected for their properties and characteristics (gas trapping, oil absorption, oil adsorption, particulate filtering). Moreover, certain embodiments of the fluid delivery system can be outfitted with multiple gas trapping filters located in different sections of the fluid flow path. For example, one filter component can be positioned at or near the fluid reservoir, and another filter component can be positioned at or near the distal end of the fluid infusion set. These and other practical implementations are contemplated by this disclosure.

As mentioned above, the fluid conduit assembly that carries the gas trapping filter can be realized in a number of different forms. For example, the fluid conduit assembly may include or be realized as a fluid connector, where the gas trapping filter is integrated in the fluid connector. In this regard, FIG. 5 is an exploded and partially phantom view of a fluid connector assembly 500 suitable for use with a fluid conduit assembly. The illustrated embodiment of the fluid connector assembly 500 functions to physically and fluidly couple an upstream section of tubing 502 to a downstream section of tubing 504. The fluid connector assembly 500 includes a first connector 506 (which is physically and fluidly coupled to the upstream section of tubing 502) that mates with a second connector 508 (which is physically and fluidly coupled to the downstream section of tubing 504). The first connector 506 includes a hollow needle 510 that provides a fluid flow path from the upstream section of tubing 502. The second connector 508 includes a septum 512 that receives the hollow needle 510 when the first connector 506 engages the second connector 508. When the two connectors 506, 508 are engaged and locked together, the medication fluid can flow from the upstream section of tubing 502, through the hollow needle 510, and into the downstream section of tubing 504.

One or both of the connectors 506, 508 can be provided with a gas trapping filter having the characteristics and functionality described previously. For this particular embodiment, a unitary gas trapping filter 516 is integrated in the second connector 508. The gas trapping filter 516 is located within the body of the second connector 508, and it resides downstream from the septum 512. During a fluid delivery operation, the medication fluid exits the hollow needle 510, enters the second connector 508 (e.g., into a space that is upstream from the gas trapping filter 516), and is forced through the gas trapping filter 516 before it passes into the downstream section of tubing 504.

By positioning a gas trapping filter inside the connector, particularly the connector adjacent the downstream tubing 504 it is able to retain and/or absorb / adsorb any small amounts of air that may be introduced by closure of the connector, thus preventing this air from entering the downstream tubing 504.

As another example, a fluid conduit assembly configured as described herein may include or be realized as an infusion set for a fluid infusion pump, where the gas trapping filter is integrated in the infusion set. In this regard, FIG. 6 is an exploded perspective view of a fluid conduit assembly that is realized as a cap or a connector assembly 600 for a fluid reservoir. In this regard, the connector assembly 600 is generally configured as described above for the connector assembly 214 shown in FIG. 2. Accordingly, the connector assembly 600 may be provided as component of a disposable infusion set.

The illustrated embodiment of the connector assembly 600 generally includes, without limitation: a body section 602; a flow path defined in the body section 602; a length of tubing 604 extending from the body section 602; and a gas trapping filter 606. FIG. 6 depicts the body section 602 separated into two constituent parts: a lower body section 602a; and an upper body section 602b. The lower body section 602a can be affixed to the upper body section 602b (for example, by sonic welding or using an adhesive) after installing the gas trapping filter 606 into a retaining cavity 610 formed within the lower body section 602a. In alternative embodiments, the body section 602 can be fabricated as a one-piece component by molding a suitable material while encapsulating the gas trapping filter 606 inside the body section 602.

The lower body section 602a is suitably configured to receive a fluid reservoir, e.g., by a threaded engagement, a snap fit, tabs, or the like. The tubing 604 is physically and fluidly coupled to the upper body section 602b such that the tubing 604 is in fluid communication with the flow path. This allows the tubing 604 to carry fluid from the body section 602 during a fluid delivery operation. The flow path, much of which is hidden from view in FIG. 6, may be defined by: a hollow needle that penetrates a septum of the fluid reservoir; an internal space, chamber, or conduit of the lower body section 602a, which is upstream of the gas trapping filter 606; and an internal space, chamber, or conduit 614 of the upper body section 602b, which is downstream of the gas trapping filter 606. The flow path continues into the tubing 604, which is connected to the upper body section 602b.

The gas trapping filter 606 is secured within the body section 602 such that it is positioned in the flow path of the medication fluid. During a fluid delivery operation, the medication fluid is forced out of the fluid reservoir and into the hollow needle (not shown in FIG. 6). The distal end of the hollow needle terminates at a location that is upstream of the gas trapping filter 606. This positioning ensures that the medication fluid can be filtered and otherwise treated by the gas trapping filter 606 before it exits the connector assembly 600. As explained above, the gas trapping filter 606 is suitably configured to reduce the amount of air bubbles in the downstream medication fluid, and optionally can reduce the amount of particulates in the downstream medication fluid.

The FIG. 6 arrangement in which the gas trapping filter is configured as a round "pill" shape. In other words a cylinder axially aligned with the retaining cavity and with a diameter to height ration greater than unity. The Fig 6 arrangement has a diameter to height ration of 5:1.

The gas trapping filter of the Figure 6 arrangement can be composed of any of the materials listed above for the gas trapping filter of the Figure 4 arrangement, or any combination thereof.

An advantage of the FIG. 6 arrangement is that the gas trapping filter presents a large surface area in both the upstream and the downstream directions than the cross-sectional area of the tubing 604. With this configuration the filter has a large working area, and also a large capacity to hold gas and, if required, particulates. As the filter is part of a disposable part of the apparatus as a whole, i.e. the cap or connector assembly for the fluid reservoir which is, in turn, connected via a tubing to the infusion unit, the device need only have a fairly short life, thus no provision need be made for the removal of the gas collected or of any particulate material that may have accumulated.

Another advantage is the filter is situated at a position in the flow path where a high wall rigidity is provided as the cap needs to be manually gripped and turned to undo the reservoir from the infusion pump. Thus a greater diameter can be provided, and the gas trapping filter can be housed within this manual grip section, thus protecting it from damage.

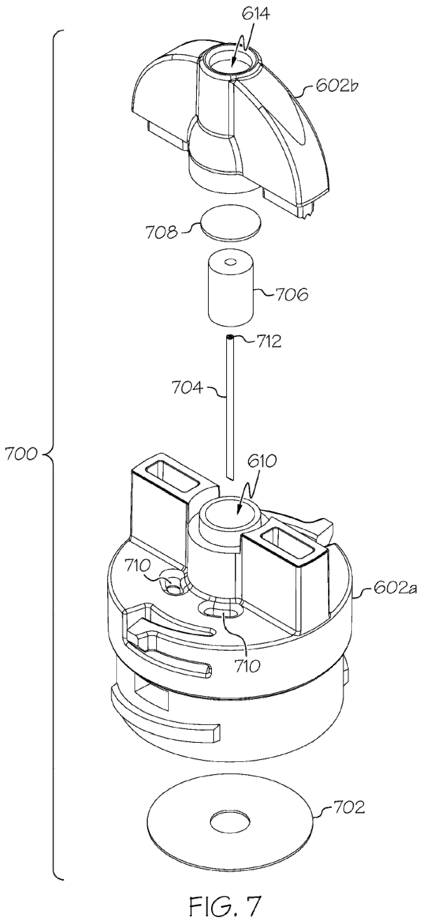

FIG. 7 is an exploded perspective view of another embodiment of a fluid conduit assembly 700 that is realized as a cap for a fluid reservoir. The assembly 700 shares some elements and features with the assembly 600 and, therefore, common elements and features will not be redundantly described here in the context of the assembly 700. As mentioned previously, the connector assembly 700 may be provided as component of a disposable infusion set.

The illustrated embodiment of the connector assembly 700 generally includes, without limitation: a body section 602 (having a lower body section 602a and an upper body section 602b); a venting membrane 702; a hollow needle 704; a gas trapping filter 706; and a reservoir membrane 708. These components can be assembled together in the manner generally described above for the assembly 600.

The venting membrane 702 can be affixed to the upper interior surface of the lower body section 602a such that the venting membrane 702 covers one or more vent holes 710 formed in the top portion of the lower body section 602a. The vent holes 710 facilitate venting of the reservoir chamber that resides in the housing of the fluid infusion device (see, for example, FIG. 2). The hollow needle 704 can be affixed to the lower body section 602a such that the downstream end 712 of the hollow needle 704 resides below or within the gas trapping filter 706 after the fluid conduit assembly 700 is fabricated. The positioning of the downstream end 712 is important to ensure that the medication fluid is forced through the gas trapping filter 706 during fluid delivery operations. The reservoir membrane 708 can be affixed within a cavity formed in the upper body section 602b (the cavity is hidden from view in FIG. 7). The reservoir membrane 708 is at least partially hydrophilic to allow the medication fluid to pass during fluid delivery operations.

The gas trapping filter 706 is secured within the body section 602 such that it is positioned in the flow path of the medication fluid. For the illustrated embodiment, the gas trapping filter 706 may be positioned between the reservoir membrane 708 and the downstream end 712 of the hollow needle 704. The gas trapping filter for example the filter 706 in the Figure 7 arrangement or the filter 606 in the Figure 6 arrangement may be is realized as a foam, sponge, or felt fiber composite material. Although not always required, the material used for the gas trapping filters 606 and 706 may include, without limitation: polyvinyl acetate (PVA); polyvinyl alcohol; polyester (PET); polycarbonate; polyurethane; polyethyl sulfone; collagen; polycaprolactone; or any combination thereof. In accordance with certain embodiments, a felt-based gas trapping filters 606 and 706 have a pore size within the range of about one to 1 00 microns, and preferably within the range of about 20 to 40 microns. A sponge-based gas trapping filter 606 or 706 may have a pore size within the range of about 20 to 1000 microns. Regardless of their composition and configuration, the gas trapping filters 606 and 706 are suitably configured to reduce the amount of air bubbles in the downstream medication fluid, and preferably also to reduce the amount of particulates in the downstream medication fluid.

The FIG. 7 arrangement takes advantage of the relatively tall body of the wing-like extension of the reservoir cap, which is provided for the user to grip and turn the cap. In the FIG. 7 arrangement the gas trapping filter is configured as a cylinder co-axial with the flow path. This arrangement addresses the problem of how to accommodate a relatively large and, hence, low resistance gas trapping filter with a high gas retaining capacity. The solution here is to configure the gas trapping filter 706 as a tall cylinder, in other words the diameter to height ratio should be less than or equal to unity. For example the diameter to height ration in the Fig 7 arrangement is 83%. To improve through flow the filter may be fed from a central cavity and exhausted partially via its outer curved surface. In the Fig. 7 arrangement the additional membrane 708 ensures that only medicinal fluid passes into the upper body section, the gas trapping filter 706 retaining any bubbles.

While at least one exemplary embodiment has been presented in the foregoing detailed description, it should be appreciated that a vast number of variations exist. It should also be appreciated that the exemplary embodiment or embodiments described herein are not intended to limit the scope, applicability, or configuration of the claimed subject matter in any way. Rather, the foregoing detailed description will provide those skilled in the art with a convenient road map for implementing the described embodiment or embodiments. It should be understood that various changes can be made in the function and arrangement of elements without departing from the scope defined by the claims, which includes known equivalents and foreseeable equivalents at the time of filing this patent application. The following numbered paragraphs set out embodiments forming part of the present disclosure.
Paragraph 1. A fluid conduit assembly for delivery of a medication fluid, the fluid conduit assembly comprising:
   a structure (104, 204, 308,404) defining a flow path (406) for the medication fluid; and
   a gas trapping filter (402, 606, 706) coupled to the structure and positioned in the flow path to filter particulates from the medication fluid and retain gas bubbles from the medication fluid.
Paragraph 2. The fluid conduit assembly of paragraph 1, wherein the gas trapping filter (402, 606, 706) comprises an at least partially hydrophilic membrane.
Paragraph 3. The fluid conduit assembly of paragraph 1 or 2, wherein the gas trapping filter has a pore size within the range of 0.45 to 5.00 microns.
Paragraph 4. The fluid conduit assembly of paragraph 1, wherein the gas trapping filter (402, 606, 706) comprises a partially hydrophilic sponge, felt, or fiber composite material.
Paragraph 5. The fluid conduit assembly of paragraph 1, wherein the gas trapping filter (402, 606, 706) comprises a material able to adsorb or absorb silicone oil.
Paragraph 6. The fluid conduit assembly of paragraph 1, wherein:
   the gas trapping filter (402, 606, 706) comprises a drug impregnated therein; and a quantity of
   the drug is releasable into the medication fluid as the medication fluid flows through the gas trapping filter (402, 606, 706) during a fluid delivery operation.
Paragraph 7. The fluid conduit assembly of any of paragraphs 1 to 6, wherein the structure comprises a reservoir cap for a fluid infusion device.
Paragraph 8. The fluid conduit assembly of any of paragraphs 1 to 6, wherein the structure comprises an infusion set for a fluid infusion device.
Paragraph 9. The fluid conduit assembly of any of paragraphs 1 to 6, wherein the structure comprises a fluid connector.
Paragraph 10. A fluid delivery system comprising:
   a fluid infusion pump to provide a medication fluid;
   a fluid conduit assembly as defined in any preceding paragraph, wherein the flow path is connected to the infusion pump to receive said medication fluid therefrom.
Paragraph 11. The fluid conduit assembly according to any of paragraphs 1 to 9, wherein the gas trapping filter comprises an upstream component (706) consisting of an at least partially hydrophilic sponge, felt or filter composite material to retain gas bubbles from the medication fluid; and
   a downstream component (708) consisting of an at least partially hydrophilic membrane to allow only the medication fluid to exit the filter.
Paragraph 12. A cap (600, 700) for a reservoir of a medicinal fluid which provides a fluid flow path for the medicinal fluid to tubing (604) used to deliver said fluid to a user; said cap (600, 700) comprising:
   a lower body section (602a) configured to be able to engage with the reservoir and having an axial bore to conduct the medicinal fluid from the reservoir;
   an upper body section (602b) having an axial bore (614) for connection at one end to the tubing (604), the axial bore (614) of the upper body section (602b) being in fluid communication with the axial bore of the lower body section (602a) to receive the medication fluid via an internal chamber (610); wherein
   a gas trapping filter (606, 706) is housed within the internal chamber (610) and configured to retain gas bubbles thereby preventing said gas from leaving the internal chamber (610).
Paragraph 13. The cap of paragraph 12, wherein the gas trapping filter (606, 706) is adapted to have one or more of the following additional functions:
   a. filter out particulates;
   b. adsorb or absorb silicone oil;
   c. form a depot of a drug such that the drug is released into the medicinal fluid as it passes through.
Paragraph 14. The cap of paragraph 12, wherein the gas trapping filter (606, 706) is adapted to filter out particulates, and has a pore size within the range of 0.45 to 5.00 microns.
Paragraph 15. The cap of paragraph 12 or 13, wherein the gas trapping filter (606, 706) comprises a hydrophilic sponge, felt or fiber composite material.
Paragraph 16. The cap of paragraph 12, 13 or 14, wherein the gas trapping filter (606, 706) is of felt and has a pore size in the range 1-100 micrometres, and preferably 20 to 40 micrometres.
Paragraph 17. The cap of paragraph 12, 13 or 14, wherein the gas trapping filter (606, 706) is of sponge with a pore size in the range 20 micrometres to 1 millimetre.
Paragraph 18. The cap of any of paragraphs 12 to 17, wherein the lower body section (602a) and the upper body section (602b) are integral with one another.
Paragraph 19. The cap of any of paragraphs 12 to 18, wherein the lower body section (602a) includes an upstream hollow needle disposed axially within the axial bore and in fluid communication with the internal chamber (610) upstream of the gas trapping filter for piecing a septum in the reservoir and conducting the medicinal fluid therefrom to the internal chamber (610).
Paragraph 20. The cap of any of paragraphs 12 to 19, wherein the gas trapping filter (606, 706) comprises a discrete cylindrical component axially disposed in the internal chamber and having a ratio of diameter to height of greater than unity, preferably 5: 1 or greater.
Paragraph 21. The cap of paragraph 20, wherein the gas trapping filter (606) is held in the internal chamber (610) by a first annular flange forming part of the lower body section (602a), and second annular flange forming part of the upper body section (602b), at least one of said annular flanges also sealing against margins of respective major surfaces of the gas trapping filter (606) ensuring that the flow of medication fluid is through a central region of the filter.
Paragraph 22. The cap of any of paragraphs 12 to 19, wherein the gas trapping filter (706) comprises a discrete cylindrical component axially disposed in the internal chamber (610) and having a ratio of diameter to height of less than or equal to unity.
Paragraph 23. The cap of paragraph 22, wherein there is provided a hydrophilic membrane (708) separating the internal chamber (610) from the bore of the upper body section (602b).
Paragraph 24. The cap of any of paragraphs 12 to 23, wherein the upper body section (602b) has a manual grip radially outwardly from the gas trapping filter, enabling the cap (600) to readily be turned by hand about its longitudinal axis.
Paragraph 25. A fluid delivery system for delivering a medicinal fluid to a user comprising: an infusion pump (202) including a reservoir of the medicinal fluid, said reservoir having a cap (214) in accordance with any of paragraphs 12 to 24;
   a length of tubing (210, 604) connected at a proximal end to the axial bore (614) of the upper body section (602b) of the cap (600,700), and at a distal end to an infusion unit (212) having a canula to enable fluid from the reservoir to be infused into the body of the user during operation of the pump. (202).
Paragraph 26. A disposible fluid delivery assembly comprising a reservoir of the medicinal fluid, said reservoir having a cap (214, 600, 700) in accordance with paragraph 24, the cap (214, 600, 700) further being provided with thread or bayonet fitting for detachably mounting the reservoir in an infusion pump, whereby the assembly can be detached for disposal by turning the cap (214, 600, 700) via said manual grip.
Paragraph 27. A fluid connector assembly (500) for a medication fluid comprising:
   a first connector (506) having a bore for connection at one end to an upstream tubing (502);
   a second connector (508) having a bore for connection at one end to a downstream tubing (504); the first and second connectors (506, 508) being detachably couplable, such that when coupled the respective bores are in fluid communication allowing flow of the medication fluid from the upstream tubing (502) to the downstream tubing (504) via the said coupled bores;
   wherein one of the first and second connectors (506, 508) contains a hollow needle (510) and the other contains a septum (512), and when the first and second connectors (506, 508) are coupled the needle pierces the septum thereby providing the said fluid
   communication between the bores of the said respective connectors (506, 508);
   a gas trapping filter (516) disposed in the bore of the second connector (508) to retain gas bubbles in the medication fluid and hinder such bubbles from traveling with the medication fluid into the downstream tubing (504).
Paragraph 28. The fluid connector assembly of paragraph 27, wherein the gas trapping filter is adapted to have one or more of the following additional functions:
   a. filter out particulates;
   b. adsorb or absorb silicone oil;
   c. form a depot of a drug such that the drug is released into the medicinal fluid as it passes through.
Paragraph 29. The fluid connector assembly according to paragraph 27 or 28, wherein the gas trapping filter (516) is molded inside the bore of the second connector (508) forming an integral component therewith.
Paragraph 30. The fluid connector assembly of paragraph 27, 28 or 29 further including a second gas trapping filter disposed in the bore of the first connector (506) to detain gas bubbles in the medication fluid entering the fluid connector assembly (500) from the upstream tubing (502).
Paragraph 31. The fluid connector assembly of paragraph 30, wherein the second gas trapping filter is adapted to have one or more of the following additional functions:
   a. filter out particulates;
   b. adsorb or absorb silicone oil;
   c. form a depot of a drug such that the drug is released into the medicinal fluid as it passes through.
Paragraph 32. The fluid conduit assembly of any of paragraphs 1 to 9 and 11, the delivery system of paragraph 10 or 26, the cap of any of paragraphs 12 to 24 or the connector assembly according to any of paragraphs 27 to 31, wherein the gas trapping filter is of a predominantly hydrophilic material optionally exhibiting up to 50% hydrophobicity.

Further disclosed herein is the subject-matter of the following clauses:
1. A fluid conduit assembly for delivery of a medication fluid, the fluid conduit assembly comprising:
   a structure (104, 204, 308, 404) defining a flow path (406) for the medication fluid; and
   a gas trapping filter (402, 606, 706) coupled to the structure and positioned in the flow path to filter particulates from the medication fluid and retain gas bubbles from the medication fluid;
   wherein the gas trapping filter (402, 606, 706) is fabricated from material having micro-cavities formed therein for trapping and retaining gas bubbles from the medication fluid.
2. The fluid conduit assembly of clause 1, wherein the gas trapping filter (402, 606, 706) comprises a material selected from the group consisting of polyacrylate; polyurethane; nylon; cellulose acetate; polyvinyl alcohol; polyethelene foam; polyvinyl acetate (PVA); polyester fiber felt; polyester (PET); polysulfone; polyethyl sulfone; collagen; and polycaprolactone.
3. The fluid conduit assembly of clause 1 or 2, wherein the gas trapping filter is of a predominantly hydrophilic material exhibiting up to 50% hydrophobicity.
4. The fluid conduit assembly of clause 1, 2, or 3 wherein the gas trapping filter has a pore size within the range of 0.45 to 5.00 microns.
5. The fluid conduit assembly of clause 1, wherein the gas trapping filter (402, 606, 706) comprises a material able to adsorb or absorb silicone oil.
6. The fluid conduit assembly of clause 1, wherein the gas trapping filter (402, 606, 706) comprises a drug impregnated therein; and a quantity of the drug is releasable into the medication fluid as the medication fluid flows through the gas trapping filter (402, 606, 706) during a fluid delivery operation.
7. The fluid conduit assembly of any of clauses 1 to 6, wherein the structure comprises an infusion set for a fluid infusion device.
8. The fluid conduit assembly according to any of clauses 1 to 7, wherein the gas trapping filter comprises:
   an upstream component (706) consisting of an at least partially hydrophilic sponge, felt or filter composite material to retain gas bubbles from the medication fluid; and
   a downstream component (708) consisting of an at least partially hydrophilic membrane to allow only the medication fluid to exit the filter.
9. A fluid delivery system comprising:
   a fluid infusion pump to provide a medication fluid;
   a fluid conduit assembly as defined in any preceding claim, wherein the flow path is connected to the infusion pump to receive said medication fluid therefrom.
10. A fluid connector assembly (500) for a medication fluid comprising:
   a first connector (506) having a bore for connection at one end to an upstream tubing (502);
   a second connector (508) having a bore for connection at one end to a downstream tubing (504);
   the first and second connectors (506, 508) being detachably couplable, such that when coupled the respective bores are in fluid communication allowing flow of the medication fluid from the upstream tubing (502) to the downstream tubing (504) via the said coupled bores;
   wherein one of the first and second connectors (506, 508) contains a hollow needle (510) and the other contains a septum (512), and when the first and second connectors (506, 508) are coupled the needle pierces the septum thereby providing the said fluid communication between the bores of the said respective connectors (506, 508);
   a gas trapping filter (516) disposed in the bore of the second connector (508) to retain gas bubbles in the medication fluid and hinder such bubbles from traveling with the medication fluid into the downstream tubing (504).
11. The fluid connector assembly of clause 10, wherein the gas trapping filter is adapted to have one or more of the following additional functions:
   a. filter out particulates;
   b. adsorb or absorb silicone oil;
   c. form a depot of a drug such that the drug is released into the medicinal fluid as it passes through.
12. The fluid connector assembly according to clause 10 or 11, wherein the gas trapping filter (516) is molded inside the bore of the second connector (508) forming an integral component therewith.
13. The fluid connector assembly of clause 10, 11 or 12 further including a second gas trapping filter disposed in the bore of the first connector (506) to detain gas bubbles in the medication fluid entering the fluid connector assembly (500) from the upstream tubing (502).
14. The fluid connector assembly of clause 13, wherein the second gas trapping filter is adapted to have one or more of the following additional functions:
   a. filter out particulates;
   b. adsorb or absorb silicone oil;
   c. form a depot of a drug such that the drug is released into the medicinal fluid as it passes through.
15. The fluid connector assembly according to any of clauses 10 to 14, wherein the gas trapping filter is of a predominantly hydrophilic material optionally exhibiting up to 50% hydrophobicity.

## Claims

1. A fluid delivery system comprising:
- a fluid infusion device (102, 202, 302) comprising a fluid reservoir (106),
- a fluid conduit assembly (104, 400) integrated in the fluid infusion device (102, 202, 302), and
- a gas trapping filter (402, 606, 706),
wherein the fluid conduit assembly (104, 400) comprises a structure (404) defining a flow path (406) for a medication fluid,
wherein the gas trapping filter (402, 606, 706) is positioned in the flow path such that the medication fluid passes through the gas trapping filter during fluid delivery operations, wherein the gas trapping filter (402, 606, 706) is configured to filter particulates from the medication fluid, and
wherein the gas trapping filter (402, 606, 706) is configured to physically obstruct the flow path such that the medication fluid is not diverted around the gas trapping filter (402, 606, 706).

2. The fluid delivery system of claim 1, wherein the fluid conduit assembly (104, 400) forms a part of the fluid reservoir.

3. The fluid delivery system of claim 1 or 2, wherein the gas trapping filter (402, 606, 706) is formed from a polyvinyl alcohol material.

4. The fluid delivery system of claim 3, wherein the polyvinyl alcohol material is treated to enhance hydrophilic characteristics.

5. The fluid delivery system of one of the claims 1 to 4, wherein the gas trapping filter (402, 606, 706) comprises a partially hydrophilic membrane or foam, particularly wherein the gas trapping filter has a pore size within the range of 0,45 to 5 microns.

6. The fluid delivery system of one of the claims 1 to 5, wherein the gas trapping filter (402, 606, 706) comprises a felt, particularly wherein the gas trapping filter has a pore size within the range of 1 to 100 microns, preferably within the range of about 20 to 40 microns.

7. The fluid delivery system of one of the claims 1 to 6, wherein the gas trapping filter (402, 606, 706) comprises a hydrophilic sponge, particularly wherein the gas trapping filter has a pore size within the range of 20 micrometers to 1 millimetre.

8. The fluid delivery system of one of the claims 1 to 7, wherein:
the gas trapping filter (402, 606, 706) comprises a drug impregnated therein; and
the gas trapping filter (402, 606, 706) is configured such that, during a fluid delivery operation, a quantity of the drug is released into the medication fluid as the medication fluid flows through the gas trapping filter.

9. The fluid delivery system of one of the claims 1 to 8, wherein the gas trapping filter (402, 606, 706) comprises a plurality of physically distinct elements that collectively function as the gas trapping filter.

10. The fluid delivery system of one of the claims 1 to 9, further comprising a reservoir membrane (708) positioned along the flow path, downstream of the gas trapping filter (402, 606, 706).

11. The fluid delivery system of one of the claims 1 to 10, wherein the medication fluid is insulin.
